# EUROPEAN PATENT APPLICATION

(11) **EP 3 875 132 A1**
(43) Date of publication of application: **08.09.2021**
(21) Application number: 20205172.8
(22) Date of filing: 02.11.2020
(51) Int. Cl.: A61M 5/315

(54) **INSULIN-DOSAGE DETECTION DEVICE**

(30) Priority: 02.03.2020 TW 109106667
(71) Applicant: Quanta Computer Inc., Taoyuan City 333 (TW)
(72) Inventor: CHENG, Kai-Ju, 333 Taoyuan City (TW); SHEN, Huan-Pin, 333 Taoyuan City (TW); WU, Huan-Tang, 333 Taoyuan City (TW)
(74) Representative: Michalski Hüttermann & Partner Patentanwälte mbB

(57) **Abstract**

An insulin-dosage detection device is arranged on a rotation member of an insulin injection pen having an injection button. The rotation variation of the rotation member corresponds to the injection dosage of the insulin injection pen. The insulin-dosage detection device has a circuit, a detection button, and a processor. The circuit, arranged on the rotation member of the insulin injection pen, is configured to output electronic signals in response to rotation of the rotation member. The detection button is connected to the injection button. The rotation member of the insulin injection pen rotates in response to the detection button and the injection button being pressed. The circuit rotates along with the rotation member. The processor, coupled to the circuit, calculates the rotation variation of the rotation member according to a first electronic signal and a second electronic signal respectively output from the circuit before and after the rotation of the circuit.

## Description

### BACKGROUND OF THE INVENTION

### Field of the Invention

The present invention relates to an insulin-dosage detection device, and in particular, the device detects the insulin dosage according to the variation of electronic signals.

### Description of the Related Art

In terms of existing technology, after a patient uses an insulin injection pen to inject insulin on his own, the patient will often only manually record the injected dosage. Unfortunately, this manual recording method is susceptible to errors and omissions. In some cases, patients may not remember whether they have injected insulin or not, and there may be the risk of repeated injection. Therefore, how to record a patient's insulin dosage is effectively an important issue.

### BRIEF SUMMARY OF THE INVENTION

Accordingly, the present invention provides an insulin-dosage detection device. The insulin-dosage detection device uses the variation of the electronic signals output from a circuit to automatically detect the dosage administered by an insulin injection pen, and transmits the detected data back to a medical center in order to maintain accurate recordkeeping of the patient's insulin dosage.

An embodiment of the present invention provides an insulin-dosage detection device. The insulin-dosage detection device is arranged on a rotation member of an insulin injection pen. The insulin injection pen has an injection button and the rotation member, wherein the rotation variation of the rotation member corresponds to the injection dosage of the insulin injection pen. The insulin-dosage detection device comprises a circuit, a detection button, and a processor. The circuit is connected to the rotation member of the insulin injection pen. The circuit is configured to output electronic signals in response to rotation of the rotation member. The detection button is connected to the injection button of the insulin injection pen. If the detection button is pressed, the injection button is correspondingly pressed to make the rotation member of the insulin injection pen rotate. If the rotation member of the insulin injection pen rotates, the circuit correspondingly rotates. The processor is coupled to the circuit. The processor obtains the rotation variation of the rotation structure according to first and second electronic signals output from the circuit before and after the rotation of the circuit.

In one embodiment of the present invention, the circuit generates a first voltage before the rotation of the rotation member of the insulin injection pen, and the circuit generates a second voltage after the rotation of the insulin injection pen. The processor calculates the real injection dosage according to the voltage variation between the first voltage and the second voltage.

In one embodiment of the present invention, the circuit comprises an impedance network, and a plurality of metal pad pairs coupled to the impedance network.

In one embodiment of the present invention, the insulin-dosage detection device further comprises a case and a metal contact. The case covers the circuit and the processor. The metal contact is fixed to the case. When the circuit rotates, each of the metal pad pairs can be connected to the metal contact respectively. When different one of the metal pad pairs is connected to the metal contact, the circuit can generate different voltage.

In one embodiment of the present invention, the insulin-dosage detection device further comprises a power switch. The power switch is provided for users to press or touch in order to turn on or turn off the insulin-dosage detection device.

In one embodiment of the present invention, the insulin-dosage detection device further comprises a power indictor. The power indicator is coupled to the power switch. When the insulin-dosage detection device is turned on, the power indicator glows. When the insulin-dosage detection device is turned off, the power indicator is off.

In one embodiment of the present invention, the insulin-dosage detection device further comprises a wireless communication unit. The wireless communication unit is coupled to the processor. The wireless communication unit transmits the real injection dosage calculated by the processor to a personal computer, a smartphone, or an electronic device that has a communication function.

In one embodiment of the present invention, the wireless communication unit is a Bluetooth communication unit.

In one embodiment of the present invention, the insulin-dosage detection device further comprises a wireless communication unit. The wireless communication unit is coupled to the processor. The wireless communication unit transmits the real injection dosage calculated by the processor to a server of a medical center.

In one embodiment of the present invention, the wireless communication unit is a long-distance mobile communication unit.

### BRIEF DESCRIPTION OF THE DRAWINGS

The present invention can be more fully understood by reading the subsequent detailed description and examples with references made to the accompanying drawings, wherein:
FIG. 1 shows a schematic diagram of an insulin-dosage detection device and an insulin injection pen according to one embodiment of the present invention;
FIG. 2 shows a schematic assembly of the insulin-dosage detection device and the insulin injection pen according to one embodiment of the present invention; and
FIG. 3 shows a detailed structure of the insulin-dosage detection device according to one embodiment of the present invention.

### DETAILED DESCRIPTION OF THE INVENTION

The following description is use to show general principles of the invention and should not serve as limitations. The scope of the invention is best determined by reference to the appended claims.

An embodiment of the present invention provides an insulin-dosage detection device. The insulin-dosage detection device uses the variation of the electronic signals to detect the dosage of insulin injection pen, and transmits the detected data back to the medical center in order to solve the missing or wrong records of the patient's insulin dosage.

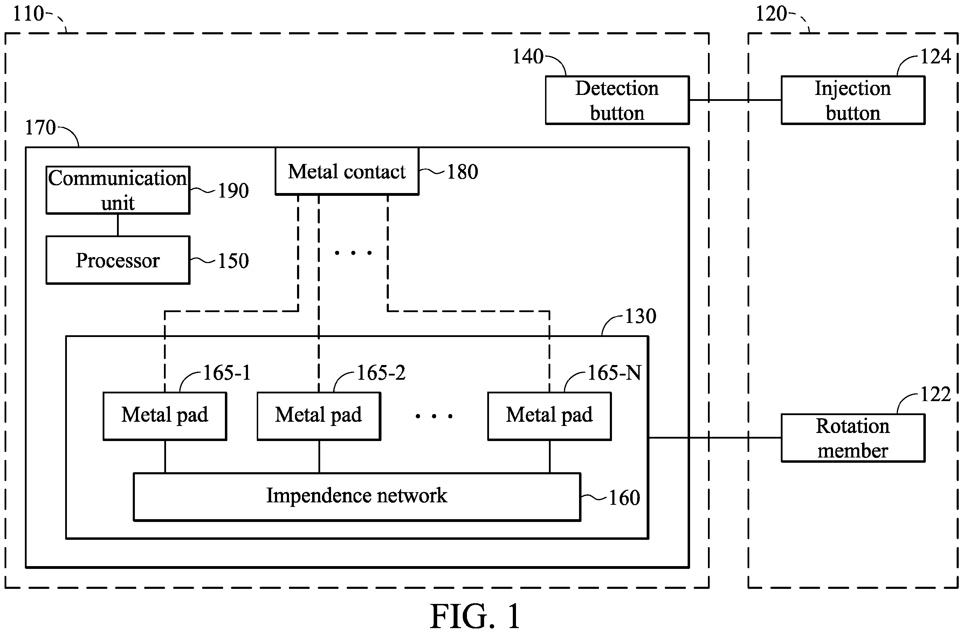

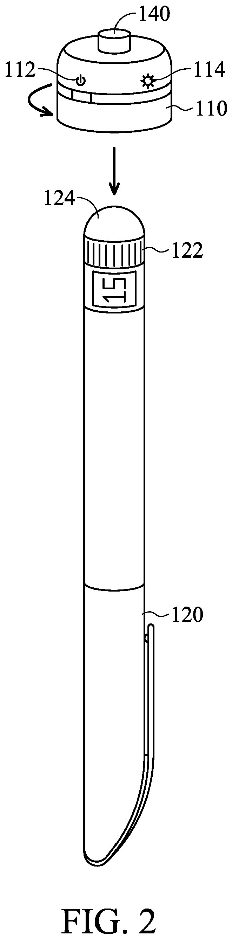

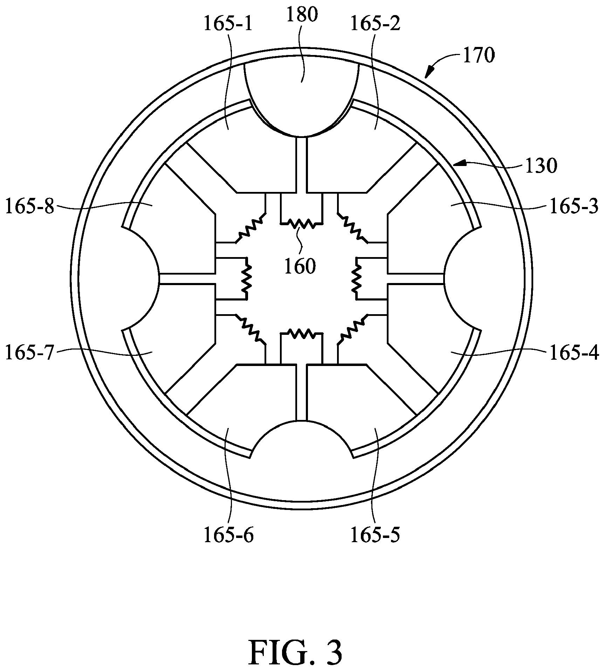

FIG. 1 shows a schematic diagram of an insulin-dosage detection device and an insulin injection pen according to one embodiment of the present invention. FIG. 2 shows a schematic assembly of the insulin-dosage detection device and the insulin injection pen according to one embodiment of the present invention. FIG. 3 shows a detailed structure of the insulin-dosage detection device according to one embodiment of the present invention. In one embodiment of the present invention, an insulin-dosage detection device 110 detects the real injection dosage of an insulin injection pen 120. The insulin injection pen at least has an injection button 124 and a rotation member 122. The rotation variation of the rotation member 122 corresponds to the injection dosage of the insulin injection pen 120. That is, users can adjust the injection dosage through rotating the rotation member 122, for example, adjusting the insulin dosage for 5ml or 10ml. The higher the injection dosage is, the more the adjustment rotation variation is. After adjusting the injection dosage through rotating the rotation member 122, users can press the injection button 124 to inject the insulin.

Referring to FIG. 2, the insulin-dosage detection device 110 is mounted to the rotation member 122 of the insulin injection pen 120. Referring to FIG. 1, the insulin-dosage detection device 110 comprises a circuit 130. The circuit 130 comprises an impedance network 160, a plurality of metal pads 165-1∼165-N. The insulin-dosage detection device 110 further comprises a detection button 140, a processor 150, a case 170 covering the circuit 130 and the processor 150, and a metal contact 180 disposed to the case 170. In some embodiments, the insulin-dosage detection device 110 can detachably mount to the insulin injection pen 120. The circuit 130 can be connected or embedded to the rotation member 122 of the insulin injection pen 120 such that the circuit 130 will rotate in response to rotation of the rotation member 122 when the injection button 124 is pressed through the detection button 140.

As illustrated in FIG. 2, the insulin-dosage detection device 110 comprises a power switch 112, which is provided for users to press or touch to turn on or turn off the insulin-dosage detection device 110. The insulin-dosage detection device 110 further comprises a power indicator 114 coupled to the power switch 112. When the insulin-dosage detection device 110 is turned-on, the power indicator 114 glows. When the insulin-dosage detection device 110 is turned-off, the power indicator 114 is off.

In order to detect the real injection dosage of the insulin injection pen 120 efficiently, in the present invention, the circuit 130 of the insulin-dosage detection device 110 is connected to the rotation member 122 of the insulin injection pen 120. When the circuit 130 rotates along with the rotation member 122, the circuit 130 can output different electronic signals corresponding to different rotation variations (positions or angles) of the rotation member 122. The rotation variation of the rotation member 122 can be obtained according to two different electronic signals output from the circuit 130 before and after the rotation of the circuit 130 respectively, thereby detecting the injection dosage of the insulin injection pen 120.

The detection button 140 of the insulin-dosage detection device 110 is connected to the injection button 124 of the insulin injection pen 120. Since the detection button 140 is connected to the injection button 124 as illustrated in FIG. 2, if the detection button 140 is pressed, correspondingly, the injection button 124 is also pressed. After pressing the detection button 140, the rotation member 122 of the insulin injection pen 120 rotates and the insulin-injection pen 120 injects insulin. When the rotation member 122 of the insulin-injection pen 120 rotates, correspondingly, the circuit 130 of the insulin-dosage detection device 110 also rotates. It is noted that the case 170 will not rotate along with the circuit 130.

In one embodiment of the present invention, the circuit 130 comprises an impedance network 160 and a plurality of metal pad pairs 165-1, 165-2 ..., 165-(N-1) 165-N. The metal pad pairs 165-1∼165-N are coupled to the impedance network 160. Referring to FIG. 3, the insulin-dosage detection device 110 comprises the case 170 covering the circuit 130, and the metal contact 180 fixed to the case 170. The metal contact 180 is a stretchable member with elasticity. When the user presses the detection button 140, correspondingly, the injection button 124 has also been pressed. The rotation member 122 of the insulin injection pen 120 rotates, the circuit 130 connected to the rotation member 122 of the insulin injection pen 120 correspondingly rotates. When the circuit 130 rotates with respect to the case 170, the metal contact 180 may touch each groove of the metal pad pairs (for example in Fig. 3, 165-1 and 165-2, 165-3 and 165-4, ..., 165-7 and 165-8) in sequence. When the metal contact 180 touches one of the metal pad pairs (for example, the metal pad pair 165-1 and 165-2), the two metal pads 165-1 and 165-2 are electrically connected. That is, when the circuit 130 rotates, each of the metal pad pairs can electrically connect the metal contact 180 respectively.

The processor 150 of the insulin-dosage detection device 110 can be a microchip unit with computer calculating function. The processor 150 is coupled to the circuit 130, which calculates the rotation variation of the rotation member 122 according to two electronic signals output from the circuit 130 before and after the rotation of the circuit 130 (and the rotation member 122) respectively. In the embodiment of this present invention, the impedance of the impedance network 60 is changed (or adjusted) when the metal contact 180 touches different metal pad pairs, and whereby the circuit 130 can generate different electronic signals with different voltages. The variation of two different voltages output from the circuit 130 before and after the rotation member 122 rotating can response the rotation variation of the rotation member 124.

In other words, for each injection, before the rotation of the rotation member 122 of the insulin injection pen 120, the circuit 130 generates a first voltage. After the rotation of the rotation member 122 of the insulin injection pen 120, the circuit 130 generates a second voltage. The processor 150 calculates the real injection dosage according to the voltage variation between the first voltage and the second voltage. In the following scenario, a user manually rotates the rotation member 122 according to the desired dosage to injection, for example, 15 milliliter, and the user does not need to record the desired injection dosage.

Next, the user configures the insulin-dosage detection device 110 on the top of the rotation member 122 of the insulin injection pen 120. At this time, the metal contact 180 touches the metal pad pairs 165-1 and 165-2. Then, the user presses the detection button 140 of the insulin-dosage detection device 110 for the insulin injection, correspondingly, the injection button 124 of the insulin injection pen 120 is also pressed. At this time, the circuit 130 and the rotation member 122 begin to rotate and the insulin injection pen 120 injects insulin. The metal contact 180 touches the metal pad pairs 165-7 and 165-8 after the rotation of the circuit 130 when the injection is finished. The processor 150 can automatically determines how many metal pad pairs are passed when the insulin injection pen 120 rotates according to the variation of the first and second voltages output by the circuit 130 before and after the rotation of circuit module 130 (and the rotation member 122). In the present embodiment, the processor 150 can know the metal contact 180 touches the pair of metal pads 165-1 and 165-2 in the beginning, and then in sequence touches the metal pad pairs 165-3 and 165-4, the metal pad pairs 165-5 and 165-6, and finally touches the metal pad pairs 165-7 and 165-8. Since the rotation member 122 of the insulin injection pen 120 and the circuit 130 of the insulin-dosage detection device 110 are connected or mounted together, the circuit 130 will rotates along with the rotation member 122. If the processor 150 detects that the metal contact 180 touches three metal pad pairs in sequence (through a detected voltage variation between the first and second voltages), correspondingly, the processor 150 will obtain the rotation variation of the rotation member 122. For example, in the present embodiment, if the rotation variation is three-quarter circle, every rotation of 90 degree corresponds to 5 milliliter, and thus total rotation of 270 degree corresponds to 15 milliliter. By this way, the injection dosage of the insulin can be obtained.

In one embodiment of the present invention, the insulin-dosage detection device 110 further comprises a wireless communication unit 190, which is coupled to the processor 150. The wireless communication unit 190 transmits the calculated real injection dosage to a personal computer, a smartphone, or an electronic device that has a communication function. The wireless communication unit 190 can be a Bluetooth communication unit with short-range communication function. In other words, the processor 150 transmits the calculated real insulin injection dosage to an electronic device, for example a smartphone, with communication function through the Bluetooth protocol at first. Then the smartphone transmits the calculated dosage to a remote medical center in order to provide these results to the medical staffs to evaluate or monitor.

In another embodiment, the wireless communication unit 190 of the insulin-dosage detection device 110 is a long-distance mobile communication unit, for example, communication chips of 3G, 4G, or 5G technology. The calculated results are transmitted to the remote medical center in order to provide these results to the medical staffs to evaluate or monitor.

The insulin-dosage detection device of the present invention automatically detects the real insulin injection dosage of patients in order to solve the missing records or wrong records of the patient's insulin dosage efficiently. The insulin-dosage detection device can directly or indirectly transmits the real insulin injection dosage to a server of the remote medical center. The staff of hospitals can easily obtain the real insulin injection dosage of patients to do follow-up evaluations. Therefore, the medical quality can be greatly improved.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation to encompass all such modifications and similar arrangements.

## Claims

1. An insulin-dosage detection device, arranged on a rotation member of an insulin injection pen, wherein the insulin injection pen has an injection button and a rotation member, a rotation variation of the rotation member corresponds to an injection dosage of the insulin injection pen, the insulin-dosage detection device comprising:
a circuit, arranged on the rotation member of the insulin injection pen and configured to output electronic signals in response to rotation of the rotation member;
a detection button, connected to the injection button of the insulin injection pen, the rotation member of the insulin injection pen rotating in response to the detection button and the injection button being pressed, and the circuit rotating along with the rotation member; and
a processor, coupled to the circuit, obtaining the rotation variation of the rotation member according to a first electronic signal and a second electronic signal respectively output from the circuit before and after the rotation of the circuit.

2. The device as claimed in the previous claim, wherein the circuit generates the first electronic signal before the rotation member rotates and the circuit generates the second electronic signal after the rotation member rotates, and the processor calculates a real injection dosage of the insulin injection pen according to a voltage variation between the first electronic signal and the second electronic signal.

3. The device as claimed in any of the previous claims, wherein the circuit comprises:
an impedance network; and
a plurality of metal pad pairs, coupled to the impedance network.

4. The device as claimed in the previous claim, further comprising:
a case, covering the circuit and the processor; and
a metal contact, disposed in the case;
wherein the metal contact capable of electrically connecting one pair of the metal pad pairs when the rotation member finishes rotating; and the circuit outputs a corresponding voltage in response to the connection of the metal contact and the one pair of the metal pad pairs.

5. The device as claimed in any of the previous claims, further comprising:
a power switch, configured to turn on and turn off the insulin-dosage detection device.

6. The device as claimed in the previous claim, further comprising:
a power indicator, coupled to the power switch, wherein when the insulin-dosage detection device is turned on, the power indicator glows, and when the insulin-dosage detection device is turned off, the power indicator is off.

7. The device as claimed in any of the previous five claims, further comprising:
a wireless communication unit, coupled to the processor; wherein the wireless communication unit transmits the real injection dosage calculated by the processor to a personal computer, a smartphone or an electronic device having a communication function.

8. The device as claimed in the previous claim, wherein the wireless communication unit is a Bluetooth communication unit.

9. The device as claimed in any of the previous seven claims, further comprising:
a wireless communication unit, coupled to the processor; wherein the wireless communication unit transmits the real injection dosage calculated by the processor to a server of a medical center.

10. The device as claimed in the previous claim, wherein the wireless communication unit is a long-distance mobile communication unit.
